# EUROPEAN PATENT APPLICATION

(11) **EP 3 474 012 A1**
(43) Date of publication of application: **24.04.2019**
(21) Application number: 17197196.3
(22) Date of filing: 19.10.2017
(51) Int. Cl.: G01N 33/50, A61L 27/38, C12N 5/071, C12M 1/12

(54) **METHOD FOR THE GENERATION OF A SKIN ORGANOCULTURE MODEL AND SKIN MODEL**

(71) Applicant: Galderma Research & Development, 06410 Biot (FR)
(72) Inventor: OSMAN-PONCHET, Hanan, 06600 ANTIBES (FR)
(74) Representative: Axe PI

(57) **Abstract**

The present invention relates to a method for the *in vitro* generation of skin, in particular of a skin model, thus obtained and its use for screening and testing of candidate substances and pharmaceutical compositions.

## Description

The present invention relates to a method for the *in vitro* generation of skin, in particular of a skin model.

In a further aspect, the present invention relates to the skin model thus obtained and its use for screening and testing of candidate substances and pharmaceutical compositions.

Dermal metabolism is recognized as an important consideration in evaluating the local exposure of topically applied pharmaceutical products. Different models have been used to study the dermal metabolism of specific chemicals such as Franz cell, skin homogenates and isolated keratinocytes. The objective of this work has been to validate the use of a new human skin organoculture model to study skin metabolism using [4,¹⁴C]-Testosterone as a reference molecule.

The methodology for evaluating *in vitro* percutaneous absorption dates back over 30 years with the innovation of the Franz Cell model (1975). This model has been initially used to study the dermal absorption of specific chemicals. Despite its simplicity, this model is very inconvenient when working with radiolabeled compounds since it implies cells cleaning and radioactive decontamination. The objective of this work was to develop a fast, convenient and easy-to-handle human skin organoculture model for studying both the skin metabolism and absorption of radiolabeled compounds.

The present inventors aim to develop a skin model overcoming the above drawbacks.

Namely, the present inventors developed a method for *in vitro* generation of a skin model providing a skin model for normal skin as well as a lesional skin model for skin disorders.

A first object of the solution proposed by the invention is to provide a method for the *in vitro* generation of a skin model using permeable insert in well plate comprising the steps of:
- introducing a sample of fresh skin sample in the insert;
- placing glass cylinders on the top of the skin sample to match with the upper part;
- filling the receive chamber with the appropriate organoculture medium;
- putting the appropriate volume of the compound to be tested in the cylinder;
- keeping in cell culture incubator set at 37°C, 5% CO₂ and saturated hygrometry under gentle shaking for needed time; and
- generating the *in vitro* skin model.

Thus, a second object is a skin model comprising a permeable support as a cylindrical cell culture insert nested inside the well of a cell culture plate, a skin sample and a cylinder placed on the top of the skin sample. Such a skin model is obtainable by the method of the invention.

Finally, a third object is the uses of the skin model of the invention respectively for determining absorption and/or metabolism of compounds, for screening compounds, for measuring change in collagen synthesis, and for measuring change of gene expression after single or repeated treatment of skin samples.

The invention shall be better understood when reading the following non-restricted description, with reference to the accompanying drawings, wherein:
- Figure 1 illustrates a skin organoculture model of the invention with skin sample in a permeable insert with a glass cylinder;
- Figures 2A-2C illustrate [4,¹⁴C]-Testosterone metabolite profiles in respectively epidermis, dermis and organoculture medium after 16 hours incubation;
- Figures 3A-3C illustrate metabolite profiles of [4,¹⁴C]-Testosterone obtained in skin (dermis), liver microsomes and hepatocytes;
- Figure 4 illustrates skin absorption of [4,¹⁴C]-Testosterone obtained in human skin, after 16 hours incubation; and
- Figure 5 illustrates skin absorption of [³H]-Leucotriene C4 (LTC4) obtained in human skin, after 3, 6 and 24 hours incubation.

A new skin model has been developed using a permeable insert or support such as TRANSWELL® insert sold by Corning. It consists of a cylindrical cell culture insert nested inside the well of a cell culture plate, a skin sample and a cylinder placed on the top of the skin sample.

Preferably, the permeable support is a TRANSWELL® culture plate. The insert contains a permeable membrane at the bottom with a defined pore size. The TRANSWELL® is a permeable support or insert, which is available in three membrane materials: polycarbonate, polyester and collagen-coated polytetrafluoroethylene. Preferably, the insert is in polycarbonate. The TRANSWELL® inserts are available in a variety of pore sizes.

Fresh human skin samples were seeded dermal side down in TRANSWELL® inserts in well plate prefilled with culture medium. A cylinder was placed on the top of the skin sample to match with the upper part usually used in the diffusion cells. This cylinder is preferably in glass, but can be made in other material. It is used to limit the surface of treatment and to avoid escape of medium.

This model can be adapted to treat skin surface ranging from 0.125 cm² up to 3 cm².The skill in the art can adapt the size of glass cylinders depending on the desired surface of treatment. Cylinders whose diameter is larger can be adapted to a surface of treatment of up to 3 cm².

Preferentially, the internal diameter of cylinder used is 11 mm (treatment surface 1 cm²) for inserts in 6-well plate (insert diameter: 24 mm). The internal diameter of cylinder used is preferably 8 mm (treatment surface 0.5 cm²) for inserts in 6-well plate (insert diameter: 24 mm) or 12-well plate (insert diameter: 12 mm) and the internal diameter of cylinder used is preferably 4 mm (treatment surface 0.125 cm²) for inserts in 24-well plate (insert diameter: 6.5 mm).

This new skin model allows the use of product volumes applied more important, while avoiding any leakage of the product.

For this, in order to improve the tightness of the system with application volumes higher than 10 µL, a topical skin adhesive was used to seal the cylinder on skin surface. Preferably, the topical skin adhesive is a liquid cyanoacrylate adhesive, such as PERMABOUND® adhesive.

The skin model of the invention is obtainable by a method for the *in vitro* generation of a skin model using permeable insert in well plate comprising the steps of:
- introducing a sample of fresh skin sample in the insert;
- placing glass cylinders on the top of the skin sample to match with the upper part;
- filling the receive chamber with the appropriate organoculture medium;
- putting the appropriate volume of the compound to be tested in the cylinder;
- keeping in cell culture incubator set at 37°C, 5% CO₂ and saturated hygrometry under gentle shaking for needed time; and
- generating the *in vitro* skin model.

Some tests have been previously realized to develop and validate the model.

Fresh human skin samples (2x2 cm) were seeded dermal side down in polycarbonate TRANSWELL® inserts in 6-well plate prefilled with 2 mL culture medium. A 0.5 cm² glass cylinder was placed on the top of the skin sample to match with the upper part usually used in the diffusion cells.

To ensure the tightness of the system, skin samples were treated with various volume of gentian violet and incubations were conducted at 37°C in 5% CO₂ on an orbital shaker in a humidified incubator for 24 hours. Absorbance of gentian violet in culture medium was measured at 590 nm and lateral leakage of the dye was monitored visually. Histological analysis was performed to check morphological change of skin.

To improve the tightness of the system, a 0.5 cm² cylinder was glued onto the surface of skin sample (epidermal side) (about 2x2 cm) using a minimal amount of cyanoacrylate adhesive. Skin sample was put in Petri dish filled with PBS. The cylinder was filled with 500 µL of William's culture medium containing phenol red allowing to see any leakage of the liquid. Skin sample was maintained at 37°C in cell incubator with 5% CO₂ and saturated hygrometry during 24 hours.

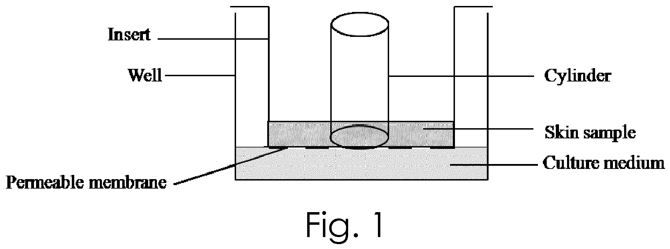

Figure 1 illustrates the new skin organoculture model with skin sample in the permeable insert with the glass cylinder.

Using gentian violet:
Whatever the application volume used, no percutaneous absorption of the dye was measured in the culture medium. Using 5 and 10 µL application volumes, no lateral diffusion of the gentian violet was observed outside the cylinder while using 20 and 30 µL application volumes, a lateral diffusion was observed. These results indicate that an application volume of 10 µL or less is suitable to ensure the tightness of the system. Histological analysis showed a good cytology of the skin and well-preserved overall morphology after 24 hours incubation.

The applicant has illustrated the skin treatment with gentian violet after 24h of incubation respectively with 5 and 10 µL (A) and 20 and 30 µL of gentian violet (B).

Using sealed cylinder:
Using a large application volume, 500 µL, no leackage of Williams's culture medium was observed outside the cylinder after 24 hours organoculture. This is more evidenced by the fact that the large applied volume still in the cylinder even after 24 hours. This result indicates that using a topical adhesive to seal the cylinder onto the surface of skin sample considerably improves the tighness of this model. Furtheremore, a large application volume to treat skin samples can be used.

The applicant has illustrated the sealed cylinder filled with 500 µL of William's culture medium after 24 hours of incubation.

This new *in vitro* model can be adapted to 12- and 24-well TRANSWELL® culture plate and can be used for kinetic experiments and for long incubation period. In fact, this model can be used in sterile conditions, to achieve long-term tests up to 5 days or so, many times greater than with diffusion cells (around 24 hours). Thus, this model can be also used for repeated treatment.

Furthermore, this new *in vitro* model is a simple, rapid, easy-to-handle and cost and time effective suitable way to study *in vitro* skin absorption and metabolism for radiolabeled as well as cold compounds. In addition, this new model is an ecologic model that offers the advantage to considerably reduce the radioactive waste generated by the cleaning and washing steps of the classical diffusion cells when used with radiolabeled compounds.

This model is particularly suitable for studying metabolism of compounds in different parts of the skin, and more specifically in the epidermis and dermis. As compounds, we understand ingredient, cosmetic or pharmaceutic compound and also cosmetic or pharmaceutic composition.

This model can also be used to assess the early inflammatory response by measure of the dendritic cell migration from the epidermis to the level of the underlayer on the support or insert and by measuring the level of cytokine release in the culture medium.

This model can also be used to measure change of gene expression in the skin after single or repeated (up to 5 days) treatment of skin samples with different compounds.

Treatment may be topically applied on skin surface or in culture medium or both.

This model can also be used to measure change in collagen synthesis.

Biopsies of normal skin but also lesional or non-lesional skin can be used in this model.

As normal skin, we talk about skin from healthy volonteers, lesional or non-lesional skin come from human subjects with dermatological diseases for who some areas of skin are affected and some others are not.

Lesional skin can also reproduced using lesional skin can be mimicked by techniques such as application of microperforation with a device such a DERMAROLLER® on normal skin or repeated stripping with adhesive tape (30 times).

Full thickness as well as split thickness skin samples (a part of the dermis is removed) can be used in this model. Skin samples without the underlayer of fat (hypodermis) as well as with the hypodermis can be used in this model. Freshly excised skin samples can be used in this model to study both metabolism and/or absorption of compounds, but also frozen skin samples can be used in this model if only skin absorption is needed.

### In vitro incubation

In one experiment, fresh human skin samples from two different donors (one man and one woman) were seeded in TRANSWELL® inserts in 6-well plate. Glass cylinders are placed on the top of the skin sample to match with the upper part. Receive chambers were filled with 1.5 mL of organoculture medium (Skin Long Term Culture Medium). Skin samples were treated in duplicate with 10 µL of [4,¹⁴C]-Testosterone solution (10 mM ∼ 500 µCi/mL) put in each cylinder and kept in cell culture incubator set at 37°C, 5% CO₂ and saturated hygrometry under gentle shaking for 4 and 16 hours. Metabolite profiles were analyzed by HPLC with radioactive detection in epidermis, dermis and receptor fluid.

HPLC conditions were:
Column: Lichrospher 100 RP18, 250*4.6mm, 5µm
Guard column: Lichrospher 100, RP18, 4*4mm, 5µm
Mobile phases: A: water, B: Acetonitrile
Flow rate: 1 mL/min
Gradient: 0 min 25%B; 50 min 50%B; 55 min 90%B; 70 min 90%B; 71 min 25%B; 81 min 25%B

*In vitro* skin metabolite profiles were compared with *in vitro* liver metabolite profiles obtained in a pool of human liver microsomes (50 µM, 5 minutes incubation) and human cryopreserved hepatocytes (50 µM, 1 hour incubation).

In another experiment, fresh human skin samples from two different donors (two women) were seeded in TRANSWELL® inserts in 6-well plate. Glass cylinders are placed on the top of the skin sample to match with the upper part. Receive chambers were filled with 1.5 mL of organoculture medium (Skin Long Term Culture Medium). Skin samples were treated in triplicate with 10 µL of [³H]-Leucotriene C4 (LTC4) solution (6.8 nM ∼ 10 µCi/mL) put in each cylinder and kept in cell culture incubator set at 37°C, 5% CO₂ and saturated hygrometry under gentle shaking. At 3h, 6h and 24 hours, radioactivity in receive chambers was measured by liquid scintillation counting. Two different experiments were performed at two different dates. Each experiment with one different donor.

### 1. Skin metabolism

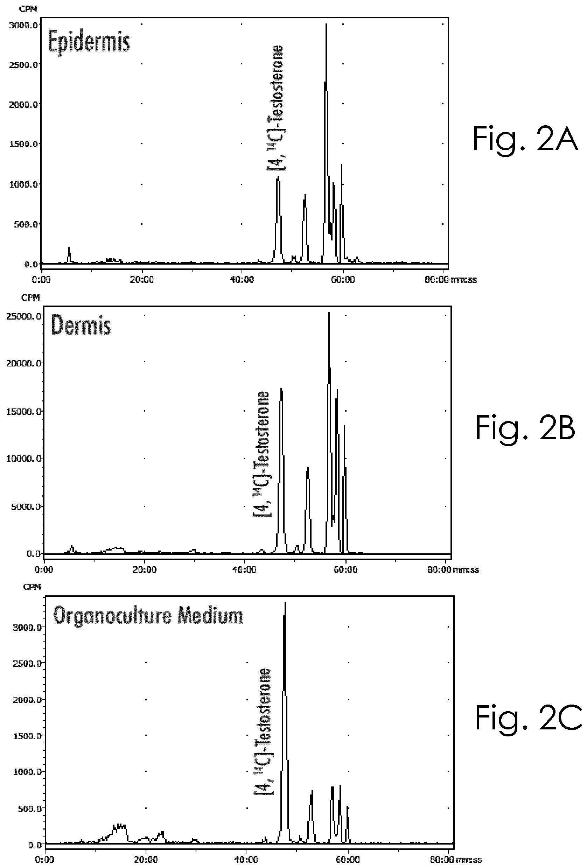

Figures 2A-2C show the [4,¹⁴C]-Testosterone metabolite profiles in respectively epidermis, dermis and organoculture medium after 16 hours incubation (man donor).

Metabolite profiles obtained after 4 hours were similar to those obtained after 16 hours. Moreover, no sex related difference was observed.

### 2. Comparison skin and liver metabolism

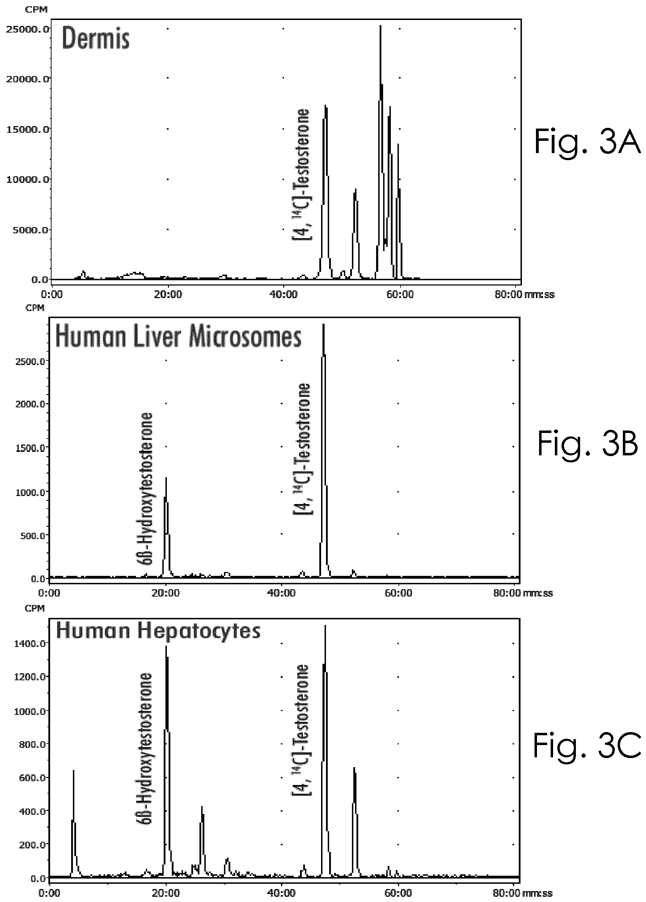

Figures 3A-3C show the metabolite profiles of [4,¹⁴C]-Testosterone obtained in respectively skin (dermis), liver microsomes and hepatocytes.

The results obtained with this new model showed that fresh excised human skin was able to metabolize efficiently [4,¹⁴C]-Testosterone. Several metabolites were observed in organoculture medium, epidermis and dermis with no sex related difference. The results also showed that skin metabolism of [4,¹⁴C]-Testosterone was clearly different from Liver metabolism. Indeed, the main metabolites of [4,¹⁴C]-Testosterone obtained in the skin resulted from reduction reactions whereas the main metabolites obtained in the liver resulted from oxidation reactions.

### 3. Skin absorption

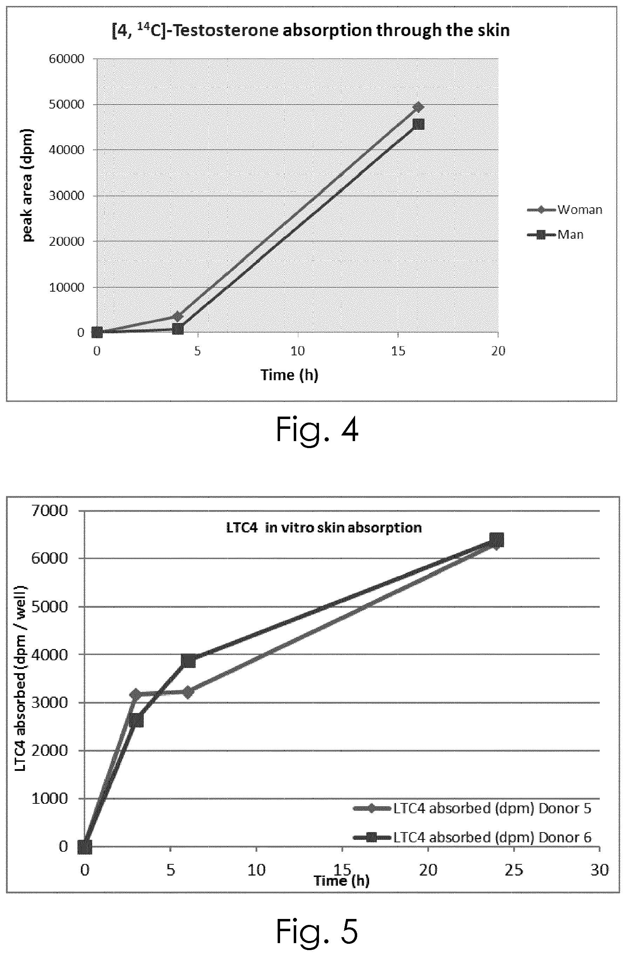

Figure 4 shows the skin absorption of [4,¹⁴C]-Testosterone obtained in human skin, after 16 hours incubation (one man and one woman donors; mean of 2 measurements per donor).

Skin absorption after 4 hours was less important to that obtained after 16 hours. Moreover, no sex related difference was observed.

Figure 5 shows the skin absorption of [³H]-Leucotriene C4 (LTC4) obtained in human skin, after 3, 6 and 24 hours incubation (2 women donors, mean of 3 measurements per donor).

Skin absorption of LTC4 increased with increasing incubation time. Moreover, no donor related difference was observed.

The results obtained with this new model showed that cutaneous absorption of two different labeled compounds can be readily measured. The results showed that the new model is very reproducible; Indeed, no sex related difference was observed with regard to testosterone skin absorption. Furthermore, with regard to LTC4 skin absorption, very reproducible results were obtained in two separate experiments using two different donors.

## Claims

1. A method for the *in vitro* generation of a skin model using permeable insert in well plate comprising the steps of:
- introducing a sample of fresh skin sample in the insert;
- placing glass cylinders on the top of the skin sample to match with the upper part;
- filling the receive chamber with the appropriate organoculture medium;
- putting the appropriate volume of the compound to be tested in the cylinder;
- keeping in cell culture incubator set at 37°C, 5% CO₂ and saturated hygrometry under gentle shaking for needed time; and
- generating the *in vitro* skin model.

2. A skin model comprising a permeable insert or support as a cylindrical cell culture insert nested inside the well of a cell culture plate, a skin sample and a cylinder placed on the top of the skin sample.

3. The skin model according to claim 2, wherein the permeable insert is a TRANSWELL® culture plate, i.e. a permeable insert with a polycarbonate, polyester or collagen-coated polytetrafluoroethylene membrane material.

4. The skin model according to claim 2 or 3, wherein the cylinder is sealed with a topical skin adhesive.

5. The skin model according to claim 4, wherein the topical skin adhesive is a cyanoacrylate adhesive.

6. The skin model according to any of claims 2 to 5 obtainable by the method according to claim 1.

7. Use of the skin model according to any of claims 2 to 6, for determining absorption and/or metabolism of compounds.

8. Use of the skin model according to any of claims 2 to 6, for screening compounds.

9. Use of the skin model according to any of claims 2 to 6, for measuring change in collagen synthesis.

10. Use of the skin model according to any of claims 2 to 6, for measuring change of gene expression after single or repeated treatment of skin samples.
